# EUROPÄISCHE PATENTSCHRIFT

(11) **EP 2 286 227 B1**
(45) Veröffentlichungstag und Bekanntmachung des Hinweises auf die Patenterteilung: **13.12.2017**
(21) Anmeldenummer: 09757475.0
(22) Anmeldetag: 29.05.2009
(51) Int. Cl.: G01N 33/535, G01N 33/543, G01N 33/58, G01N 27/49, G01N 27/403, C12Q 1/00, C12Q 1/42

(54) **VERFAHREN ZUM DETEKTIEREN VON CHEMISCHEN ODER BIOLOGISCHEN SPECIES SOWIE VORRICHTUNG ZUM DURCHFÜHREN DES VERFAHRENS**
METHOD FOR DETECTING CHEMICAL OR BIOLOGICAL SPECIES AND DEVICE FOR CARRYING OUT THE METHOD
PROCÉDÉ DE DÉTECTION D'ESPÈCES CHIMIQUES OU BIOLOGIQUES, ET DISPOSITIF DESTINÉ À CE PROCÉDÉ

(30) Priorität: 06.06.2008 DE 102008027038
(43) Veröffentlichungstag der Anmeldung: 23.02.2011
(73) Patentinhaber: Fraunhofer-Gesellschaft zur Förderung der angewandten Forschung e.V., 80686 München (DE)
(72) Erfinder: NEBLING, Eric, 25421 Pinneberg (DE); ALBERS, Joerg, 25576 Brokdorf (DE)
(74) Vertreter: Strehl Schübel-Hopf & Partner
(86) Internationale Anmeldenummer: PCT/EP2009/056616
(87) Internationale Veröffentlichungsnummer: WO 2009/147093

(56) Entgegenhaltungen:
- WO-A2-2005/073708
- WO-A2-2007/014931
- US-A1- 2007 111 202
- DAS J ET AL: "Electrochemical immunosensor using p-aminophenol redox cycling by hydrazine combined with a low background current" ANALYTICAL CHEMISTRY, Bd. 79, Nr. 7, 1. April 2007 (2007-04-01), Seiten 2790-2796, XP7909735 ISSN: 0003-2700
- LIMOGES B ET AL: "Theory and practice of enzyme bioaffinity electrodes. Chemical, enzymatic, and electrochemical amplification of in situ product detection." JOURNAL OF THE AMERICAN CHEMICAL SOCIETY, Bd. 130, Nr. 23, 20. Mai 2008 (2008-05-20) , Seiten 7276-7285, XP002545113 ISSN: 1520-5126

## Beschreibung

Die vorliegende Erfindung betrifft ein Verfahren zum Detektieren einer oder mehrerer chemischer oder biologischer Species, die entweder eine Redox-Reaktion eingehen können oder direkt oder indirekt ein Molekül freisetzen können, das eine Redox-Reaktion eingehen kann, wobei durch die genannte Redox-Reaktion generierter Strom an mindestens einer Elektrodenposition detektiert wird.

Stand der Technik sind Träger mit einer oder mehreren Messpositionen, bestehend aus einer Elektrode, an der nur eine Oxidation oder eine Reduktion eines Mediators stattfindet. Die Herstellung der Elektroden für dieses Verfahren ist einfach (z.B. Golddraht, Leiterplatten, mit Metall bedampfter Kunststoff), aber die erhaltenen Stromsignale sind oft nicht sensitiv genug.

In den letzten Jahren sind ultrafeine, interdigitale Elektrodenarrays entwickelt worden, wobei jede Messposition zwei Elektroden mit jeweils einer fingerartigen Struktur aufweist und die beiden fingerartigen Strukturen derart ineinandergreifen, dass - in der Regel parallele - Elektrodenstreifen der einen und der anderen Elektrode abwechselnd nebeneinander zu liegen kommen. Die Abstände zwischen den beiden Elektroden können im günstigen Fall deutlich unter 1 µm liegen. Ein derartiger Sensor mit Strukturbreiten der Elektroden im Sub-µm-Bereich ist beispielsweise in DE 43 18 519 A1 beschrieben. O. Niwa et al., Anal. Chem. (1993) 65, 1559-1563 konnten zeigen, dass bei der cyclovoltammetrischen Detektion von 4-Aminophenol, das durch die enzymatische Spaltung von 4-Aminophenylphosphat durch alkalische Phosphatase entstanden war, an Elektroden mit Strukturbreiten von 3-5 µm und mit einem Abstand der Finger von 2-5 µm das Messsignal gegenüber der Detektion unter Verwendung einer üblichen, einzelnen Elektrode deutlich vergrößert war.

EP 886 773 B1 offenbart ein Verfahren zum Detektieren von Molekülen oder Molekülkomplexen in einem Verdünnung- oder Lösungsmittel, wobei eine Messprobe mit einer interdigitalen Ultra-Mikroelektrodenanordnung in Kontakt gebracht wird. Für das Verfahren wird durch Anlegen eines elektrischen Potentials an die Elektrodenstrukturen ein elektrisches Wechselfeld erzeugt, und es werden die Strom- oder Potentialveränderungen gemessen, die durch in der Messprobe vorhandene oder entstehende Species verursacht werden. Dabei wird der resultierende Stromfluss hauptsächlich von den detektierten Molekülen und Molekülkomplexen im elektrodennahen Raum beeinflusst. Die Beeinflussung kann durch Diffusion, durch Anlagerung oder durch Bindung der zu messenden Species erfolgen. Die Messungen erfolgen insbesondere mit Hilfe der Impedanzspektroskopie. Das zur Detektion benutzte elektrische Feld kann durch Wechselspannung mit sehr kleinen Amplituden zwischen ca. 10 mV und 50 mV erzeugt werden; die Frequenzen können zwischen 1 mHz und 10 MHz betragen.

Die zu messenden Moleküle können auf den Mikroelektrodenflächen selbst gebunden werden. Dies kann durch physikalische (Adsorption) oder chemische Bindung erfolgen, wobei z.B. Thiolverbindungen auf Goldelektroden aufgebracht und vermessen werden. Auf den Elektroden können jedoch auch Antigene oder dergleichen aufgebracht werden, die mit einem Antikörper in der Messprobe reagieren, oder es lassen sich Hybridisierungsreaktionen in der Nucleinsäurechemie verfolgen.

Als Ausführungsbeispiel zeigt die oben genannte Druckschrift die Detektion der Anbindung von β-Galaktosidase-Streptavidin an eine S-biotinylierte Elektrodenoberfläche aus Gold. Nach der Anbindung des modifizierten Streptavidins an das Biotin wird die Änderung der Impedanz anhand eines sogenannten Nyquist-Plots gemessen, der die Störung des Dielektrikums zwischen den Elektroden durch das komplexierte Molekül zeigt und damit die vollzogene Bindung zwischen dem Biotin und dem Streptavidin-Enzym-Komplex repräsentiert. Die Ultra-Mikroelektrodenanordnung kann die Anbindung des β-Galaktosidase-Streptavidins an das Biotin außerdem amperometrisch anhand der Detektion von p-Aminophenol messen. Hierfür wird das elektrische Wechselfeld mit einem gleich bleibenden Potential überlagert, durch den die eine Elektrode auf ein Potential von +250 mV gegenüber einer Ag/AgCl-Referenzelektrode gebracht wird, mit dessen Hilfe das Aminophenol zu Chinonimin aufoxidiert wird.

WO 2007/014931 A2 offenbart ein Verfahren zur elektrochemischen Detektion eines Analyten, bei dem eine Elektrode für die Dauer einer Messphase so polarisiert wird, dass eine Reportermolekül oxidiert bzw. reduziert wird und die Elektrode für die Dauer eines Relaxationsphase so polarisiert wird, dass die oxidierte bzw. reduzierte Form des Reportermoleküls an derselben Elektrode wieder reduziert bzw. oxidiert wird, wobei der nach Abklingen der Umladung der Doppelschicht an der Elektrode gemessene Strom das Messsignal bildet.

WO 2005/073708 A2 offenbart ein Verfahren zur elektrochemischen Messung der Konzentration oder Konzentrationsänderung von redoxreaktiven Substanzen, wobei an einer Arbeitselektrode das Potenzial gepulst und abwechselnd Messphasen und Relaxationsphasen gebildet werden und am Ende des Pulses der kapazitive Strom klein gegenüber dem Faraday'schen Strom ist, und wobei die wobei die Relaxationsphasen-Pulslängen so gewählt werden, dass gegen Ende des Pulses der Konzentrationsgradient relaxiert ist, so dass zu Beginn der folgenden Messphase die Konzentrationsänderung des Mediators, verursacht durch die Messung selbst, weitgehend rückgängig gemacht wird.

US 2007/0111202 A1 offenbart ein Verfahren zur quantitativen elektrochemischen Detektion von Nukleinsäuresequenzen, bei dem elektrische Pulse an eine Arbeitselektrode angelegt werden und Signale eines Bioreportermoleküls quantitativ ausgewertet werden.

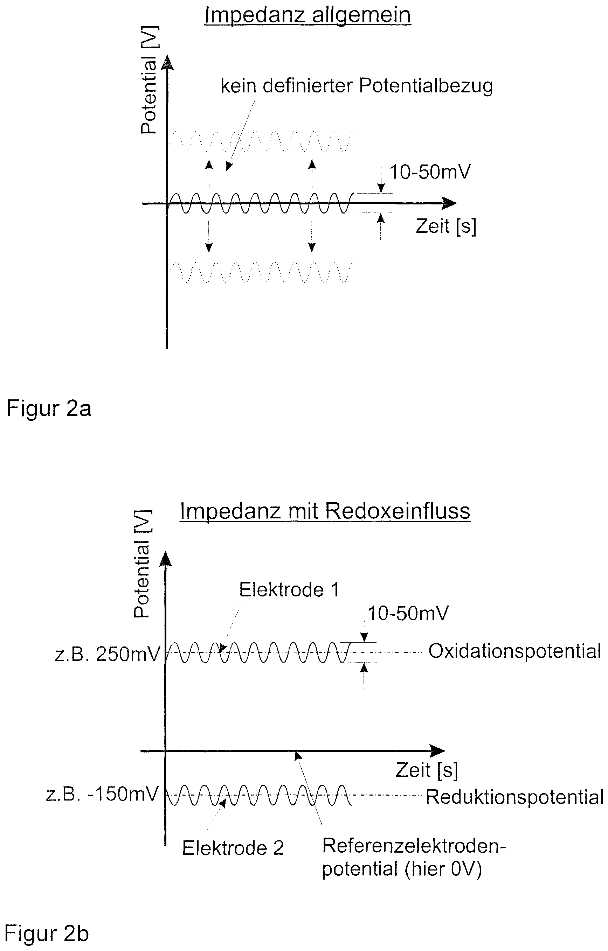

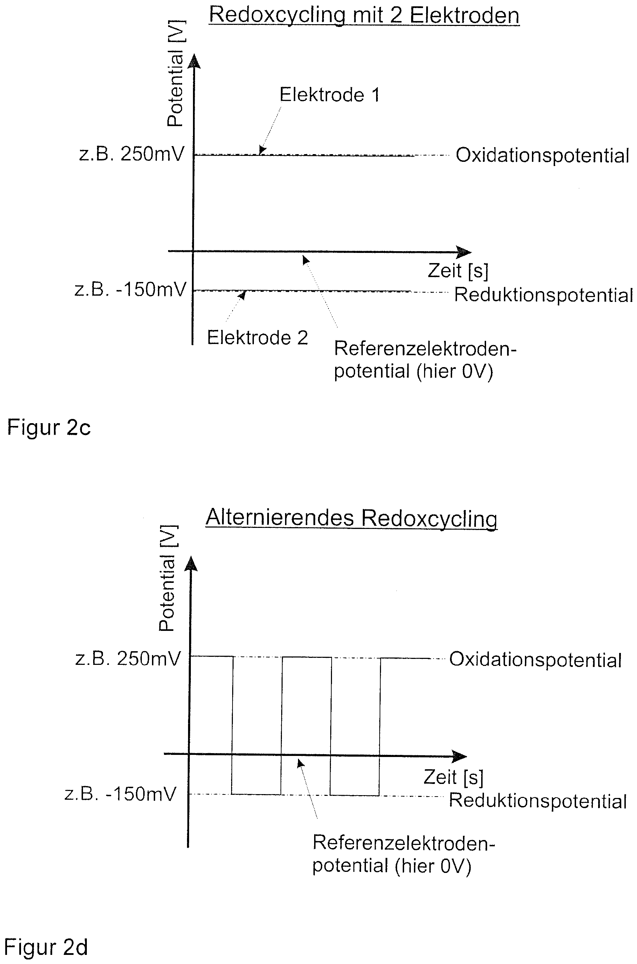

Die voranstehend geschilderten sowie weitere mögliche Messprinzipien unter Verwendung von Interdigitalelektroden seien nachfolgend genauer erläutert:
1. Mit der reinen Impedanzspektroskopie wird der komplexe Wechselstromwiderstand gemessen. Dies wird in der Elektrochemie hauptsächlich für die Charakterisierung des zwischen den Elektroden befindlichen Mediums und/oder von speziellen Belegungen der Elektrodenoberflächen genutzt, die mit Fängermolekülen (z.B. über Thiolbrücken an Goldelektroden gebundenen biologischen Fängermolekülen) besetzt sind. Mit einer Messposition, bestehend aus den genannten Interdigitalelektroden, lässt sich das Vorhandensein einer an die Fängermoleküle bindenden Species im Detektionsfluid auffinden, weil sich durch die Anbindung die Leitfähigkeit für Elektronen und/oder Protonen in der Umgebung der Elektroden ändert. Sind mehrere Messpositionen vorgesehen, so können diese gegebenenfalls mit unterschiedlichen Fängermolekülen belegt werden, die unterschiedliche Species binden, so dass mehrere Species in einem Detektionsfluid detektiert werden können. Typischerweise werden die Elektroden selbst durch die Bindung der zu detektierenden Species an den Fängermolekülen chemisch nicht verändert. Für diese Messtechnik wird eine möglichst kleine Wechselspannung (in der Regel bei oder unter 50 mV) benutzt, die generell nicht auf eine Referenz bezogen sein muss. Teilweise wird die Frequenz über sehr weite Bereiche variiert, um daraus weitere Abhängigkeiten abzulesen. Ein Diagramm, in dem diese Messung anhand der Koordinaten Potential [V] gegen die Zeit [s] aufgetragen ist, ist in **Figur 2a** dargestellt.
2. Die Impedanzspektroskopie kann zusätzlich Oxidations- und/oder Reduktionseinflüsse nutzen, wie z.B. in der obigen Druckschrift erwähnt. Wie bei der reinen Impedanzspektroskopie wird ein Wechselstromwiderstand mittels einer geringen Wechselspannung im Bereich von meist ca. 10-50 mV gemessen. Gleichzeitig wird aber mit Gleichspannung mindestens auf eine der beiden Elektroden ein definiertes Potential aufgebracht (typischerweise mittels Referenzelektrodenbezug), das gezielt eine Reduktion oder eine Oxidation eines vorhandenen oxidierbaren, reduzierbaren oder einer Redoxreaktion zugänglichen Mediators bewirkt, was wiederum Gleichströme hervorruft, die jedoch nicht unbedingt ausgewertet werden. Gemessen wird in der Regel nur deren zusätzlicher Einfluss auf die Impedanz. Ein entsprechendes Diagramm, in dem wiederum das Potential [V] gegen die Zeit [s] aufgetragen ist, ist in **Figur 2b** dargestellt.
3. Anstelle der Impedanz kann als Messprinzip bei diesen Anordnungen auch die cyclische Voltammetrie zu Einsatz kommen. Hierfür werden die Elektroden sehr langsam - in der Regel über mehrere Sekunden oder sogar Minuten hinweg - von einer vorgegebenen positiven Spannung bis zu einer vorgegebenen negativen Spannung und wieder zurück geschaltet. Das dabei erhaltene Diagramm der gemessenen Strommenge gegen die Spannung lässt Rückschlüsse auf das Vorhandensein von zu detektierenden Molekülen zwischen den Interdigitalelektroden zu. Die Cyclovoltammetrie ist im Prinzip ein sehr langsames elektrochemisches Redox-Verfahren mit entsprechendem Stoffumsatz, bei dem Oxidations- und/oder Reduktionsmaxima der jeweiligen Substanz ermittelt werden.
4. Von diesen Methoden unterscheidet sich das Redoxcycling, auch Redoxrecycling genannt. Hierfür werden die beiden Elektroden jeweils konstant mit einem Oxidations- und einem Reduktionspotential (mit Referenzelektrodenbezug) beaufschlagt, das bei oder oberhalb des Oxidations- und bei oder unterhalb des Reduktionspotentials des zu detektierenden Redoxmoleküls liegen sollte. Aufgrund der extremen Nähe der beiden Elektroden wandert das oxidierte Molekül zur gegensinnig geladenen Elektrode, wird dort reduziert und wandert wieder zurück. Gemessen werden die beiden Ströme, die direkt durch die Oxidation bzw. Reduktion der Redoxmoleküle an den Elektroden erzeugt werden. Durch die mehrfache Oxidation und Reduktion kann jedes einzelne Molekül mehrfach erfasst werden, was zu einer Verstärkung der Messsignale führt. Ein entsprechendes Diagramm, in dem wiederum das Potential [V] gegen die Zeit [t] aufgetragen ist, ist in **Figur 2c** dargestellt.

Der Nachteil an den vorgenannten Techniken liegt darin, dass die interdigitalen Elektrodenstrukturen im Sub-µm-Bereich hergestellt werden müssen, um die jeweils unterschiedlich geladenen Elektroden sehr nahe aneinander bringen zu können. Aber nur dann kann mit einer ausreichenden Empfindlichkeit gemessen werden. Dies geschieht zur Zeit mittels aufwändiger Halbleitertechnik im Reinraum, siehe z.B. E. Nebling et al., Anal. Chem. (2004) 76(3), 689-696. Ein weiterer Nachteil liegt darin, dass die große Nähe der Elektroden zueinander die Gefahr von Kurzschlüssen birgt: sollten sich Anode und Kathode bei einem Abstand von oft nur noch ca. 400 nm berühren, ist die jeweilige Messposition insgesamt unbrauchbar. Sollen mit der Elektrodenanordnung mehrere Species in einer Flüssigkeit detektiert werden, wobei mehrere Messpositionen mit unterschiedlichen Fängermolekülen belegt derselben zu untersuchenden Flüssigkeit ausgesetzt werden, so muss der ganze Test mit einer neuen Elektrodenanordnung wiederholt werden.

Um Kurzschlüsse auszuschließen, werden die Elektrodenstrukturen häufig im Substrat eingebettet. Dies ist aber natürlich mit nochmals erhöhtem Aufwand bei der Herstellung verbunden.

Es ist daher Aufgabe der Erfindung, hier Abhilfe zu schaffen und die Messung von Molekülen oder sonstigen Species wie Molekülkomplexen, Peptiden, Proteinen (insbesondere Enzymen), Antigenen, Nucleinsäuren oder Nucleinsäure enthaltenden Species etc. mit Hilfe einer Technik zu verwirklichen, die hinsichtlich der einzusetzenden Vorrichtungen zuverlässiger und weniger anspruchsvoll ist.

Gelöst wird die Aufgabe durch die Bereitstellung eines Verfahrens zum Detektieren einer oder mehrerer (chemischer oder biologischer) Species, die entweder eine Redox-Reaktion eingehen können oder direkt oder indirekt ein Molekül freisetzen können, das eine Redox-Reaktion eingehen kann, wobei durch die genannte Redox-Reaktion generierter Strom an mindestens einer Elektrodenposition detektiert wird, worin die mindestens eine Elektrodenposition, auf der oder in deren Nähe sich die zu detektierende Species befindet, mehrfach zwischen zwei unterschiedlichen Potentialen derart hin- und hergeschaltet wird, dass sie gegenüber einer Referenzelektrode Potentiale einnimmt, die in der Nähe bzw. unter / über dem Redoxpotential der genannten Species oder des genannten Moleküls liegen, so dass die genannte Species / das genannte Molekül (letzteres nach dessen Freisetzung) an oder in der Nähe dieser Elektrodenposition beim Hin- und Herschalten abwechselnd reduziert und oxidiert wird. Dabei wird der über die Zeit hinweg durch die wiederholte Reduktion und Oxidation der Species / des Moleküls gebildete Strom erfasst und die zeitliche Veränderung der Summe aus den Beträgen von Oxidations- und Reduktionsstrom ausgewertet, und ggf. mit einer Elektrodenposition verglichen, an der oder in deren Nähe sich die genannte Species oder das genannte Molekül nicht befindet. Das Erfassen und Auswerten der Strommengen und die Folgerung, dass die gesuchte Species - qualitativ oder quantitativ bestimmbar -- vorhanden ist oder nicht, erfolgt in der Regel gemäß den Merkmalen des Anspruchs 1, vorzugsweise gemäß Anspruch 2. Dabei soll unter dem Ausdruck "Positionierung einer zeitlich variierenden Menge der Species" verstanden werden, dass sich die Menge der Species auf, an oder in der Nähe der Elektroden während des Zeitraums t₁ - t₂ ändert und vorzugsweise zunimmt.

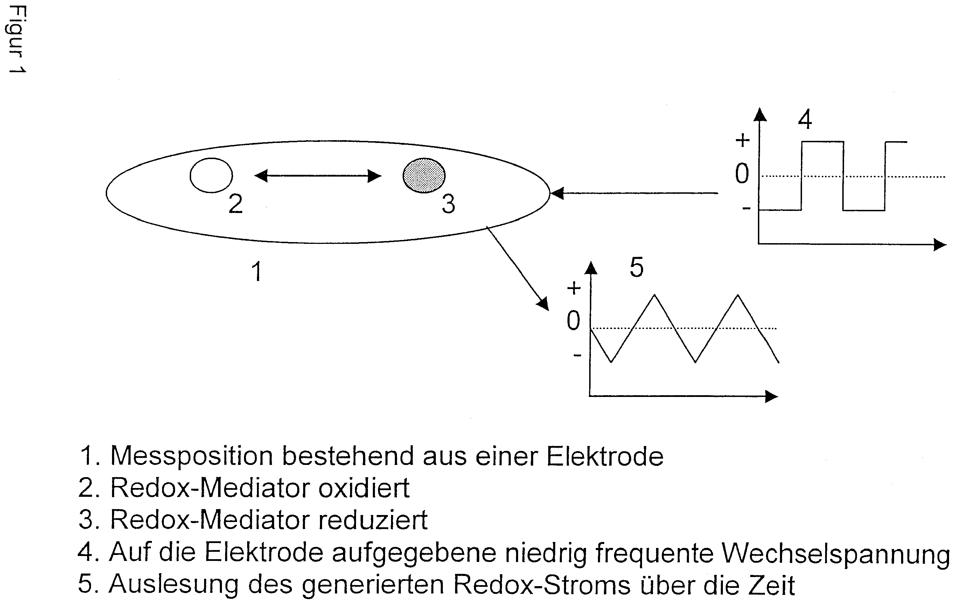

Gegenüber dem üblichen Redox-Cycling unterscheidet sich das erfindungsgemäße Verfahren also dadurch, dass keine interdigitalen Elektroden mit einer fest eingestellten Potentialdifferenz eingesetzt werden müssen, sondern dass die Spannung an nur einer Elektrode zyklisch so variiert wird, dass diese gegenüber einer Referenzelektrode abwechselnd jeweils als Kathode und Anode fungiert. Dies ist schematisch in **Figur 2d** dargestellt. Durch das - in der Regel mit z.B. 0,1 bis 10 Hz relativ niedrigfrequente - Hin- und Herschalten zwischen zwei unterschiedlichen Potentialen auf dieser Elektrode wird dann, wenn sich auf oder in ihrer Nähe eine Species oder ein Molekül befindet, das einer Redoxreaktion zugänglich ist, diese(s) durch das eine Potential oxidiert und durch das andere reduziert. Die dabei generierten Ströme - ein positiver Oxidations- und ein negativer Reduktionsstrom -werden in ihrer Summe gemessen (siehe **Figur 1****,** worin 1 eine aus einer Elektrode bestehende Messposition, 2 den oxidierten Redox-Mediator, 3 den reduzierten Redox-Mediator, 4 eine auf die Elektrode aufgegebene niedrig frequente Wechselspannung und 5 die Auslesung des generierten Redox-Stoms über die Zeit schematisch darstellen).

Ausgewertet wird dabei die zeitliche Veränderung des aufaddierten Gesamt-Stromsignals (Summe aus den Beträgen von Oxidations- und Reduktionsstrom, resultierend in einer Stromsteigerung über die Zeit), was einer Änderung der Konzentration des Redox-Mediators entspricht. Der auftretende kapazitive Umladestrom wird nicht berücksichtigt, da er unabhängig von den Redox-Strömen des Mediators ist. Er relaxiert zwar nach jeder Umladung schnell, ist aber bei jedem neuen Messzyklus nahezu identisch und ändert sich somit nicht über die Zeit.

Die Empfindlichkeit dieser Anordnung entspricht in etwa der des "herkömmlichen" Redox-Cyclings auf interdigitaler Anode und Kathode. Der komplizierte Herstellungsprozess von interdigitalen Elektroden-Arrays durch z.B. Halbleitertechnik entfällt hierbei jedoch. Eine oder mehrere Elektroden mit zyklisch wechselnden Potentialen können als eine Messposition oder als ein komplettes Array mit unterschiedlichen Messpositionen Anwendung finden.

Mit dem erfindungsgemäßen Verfahren lassen sich einerseits Moleküle (direkt) detektieren, die in einem Detektionsfluid vorhanden sind, sofern diese Moleküle eine Redoxreaktion eingehen können. Anderseits können solche Species detektiert werden, die direkt oder über Fängermoleküle an die Elektroden oder an deren Umgebung anbindbar sind und nach ihrer Anbindung direkt oder indirekt Moleküle freisetzen, die ihrerseits eine Redoxreaktion eingehen können.

Das erfindungsgemäße Verfahren lässt sich dementsprechend für die Detektion von Molekülen einsetzen, die in einer Messflüssigkeit oder einem anderen Detektionsfluid vorhanden sind und einer Redoxreaktion zugänglich sind. Unter Detektionsfluid sollen dabei Flüssigkeiten, Gele oder andere höherviskose Materialien sowie Gase verstanden werden. Es ist günstig, wenn das Detektionsfluid in einem Fluidkanal über die eine oder die mehreren Messelektroden geführt wird. Es können ggf. mehrere Detektionsfluide gleichzeitig vermessen werden, wobei jeder der Fluidkanäle über eine der Messelektroden geführt ist. Dabei kann es günstig sein, über eine der Messelektroden ein Vergleichsfluid zu führen, dessen Gehalt oder Abwesenheit an Molekül, das einer Redox-Reaktion zugänglich ist, bekannt ist. Dies erleichtert halbquantitative oder quantitative Aussagen.

So kann beispielsweise 4-Aminophenol in einer Messflüssigkeit direkt detektiert werden, indem man diese Flüssigkeit in die Nähe von Messelektroden strömen und dort verweilen lässt. Jede Elektrode wird zwischen den Redoxpotentialen von p-Aminophenol +200 mV und -350 mV mit 1,0 Hz kontinuierlich hin- und hergeschaltet. Als Bezug kann beispielsweise eine Iridium/Iridiumoxid-Referenzelektrode dienen. Eine Gegenelektrode, an beliebiger Stelle angebracht, leitet Differenzströme ab. Auch bei dieser Ausgestaltung können mehrere Elektroden vorgesehen sein, die sich jeweils in getrennten Fluidkanälen befinden.

Alternativ bindet die zu detektierende Species an die erfindungsgemäße Elektrodenstruktur und/oder deren Umgebung direkt an, beispielsweise durch sogenanntes "self-assembling", z.B. über funktionelle Gruppen auf dieser Oberfläche und/oder funktionelle Gruppen an dieser Species (z.B. binden Proteine mit SH-Gruppen an Goldoberflächen), oder die Elektrodenstruktur bzw. ihre unmittelbare Umgebung wird - über vergleichbare Mechanismen - mit einem Fängermolekül belegt, das mit der zu detektierenden Species reagiert und diese bindet oder komplexiert, wobei ein Produkt entsteht, das seinerseits ein zugesetztes Substrat in ein Molekül umwandelt, welches einer Redoxreaktion zugänglich ist (auch als Redox-Mediator bezeichnet), während das Fängermolekül bei Abwesenheit der zu detektierenden Species dasselbe Substrat nicht umwandeln kann. Der Redox-Mediator kann dabei chemisch, physikalisch oder enzymatisch generiert werden. So können z.B. Goldelektroden vorgesehen sein, auf denen ein Protein durch Adsorption gebunden ist. Ein Beispiel: Saure Phosphatase spaltet 4-Aminophenylphosphat in das Redox-Molekül 4-Aminophenol, β-Galaktosidase spaltet p-Aminophenyl-β-galaktopyranosid in dasselbe Molekül. Natürlich sind beliebige Redox-Moleküle für die vorliegende Erfindung einsetzbar; solche, die sich durch Enzyme spalten lassen, sind jedoch für die Detektion von Enzymen oder enzymhaltigen Komplexen besonders geeignet. Weitere Beispiele für brauchbare Redox-Moleküle sind Ferrocenderivate, Kaliumhexacyanoferrat(II),(III) und organische Ruthenium- und Osmiumkomplexe wie Rutheniumhexamin, Osmiumbispyridyldichlorid. Es können aber auch sonstige organische und ggf. auch anorganische Redox-Moleküle eingesetzt werden; die letzteren liegen vorzugsweise in verkapselter Form vor, wobei die Kapseln, z.B. Liposomen, z.B. eine Gruppe tragen können, die nur mit einer Kombination aus Fängermolekül und zu detektierender Species reagiert, nicht aber mit dem Fängermolekül allein. Solche Bindungsmöglichkeiten bieten z.B. sogenannten Interkalationsverbindungen, die aus der DNA-Technologie bekannt sind und nur mit doppelsträngiger Nucleinsäure, nicht aber mit einsträngiger Nucleinsäure reagieren. Der Fachmann kennt eine Reihe von Möglichkeiten, diese Techniken zu verwirklichen, siehe z.B. WO 2002/081739 oder WO 2002/082078.

Für dieses Verfahren lässt man vorzugsweise in einem ersten Schritt das die Species enthaltende Detektionsfluid über die eine oder mehreren Elektroden fließen, die bereits mit den Fängermolekülen belegt sind. Werden mehrere Elektroden verwendet, lassen sich die Elektroden mit unterschiedlichen Fängermolekülen belegen, die unterschiedliche Species binden/komplexieren können. Mit diesem Verfahren können deshalb mehrere unterschiedliche Species in einem Detektionsfluid detektiert werden, was insbesondere in der DNA-Analytik von Bedeutung ist. Selbstverständlich können die mehreren Elektroden in diesem Fall in einem gemeinsamen Fluidkanal oder in unterschiedlichen Fluidkanälen angeordnet sein.

Eine oder mehrere der als Messpositionen dienenden Elektroden können in dieser Ausgestaltung ohne Fängermoleküle bleiben. Diese eignen sich dann als Vergleichselektrode, um die jeweiligen Messwerte gegen einen geeigneten Nullwert vergleichen oder eichen zu können, was quantitative oder halbquantitative Detektionsergebnisse ermöglicht.

Die Elektroden / Messpositionen können aus einem beliebigen leitenden Material, z.B. aus einem Edelmetall wie Gold oder Platin, aber auch aus Kohlenstoffverbindungen wie Graphit oder Nanotubes bestehen; günstig ist Gold, da viele biologisch relevante Species über eine Thiolbrücke an Gold anbindbar sind. Werden die Messpositionen mit Biomolekülen belegt, können sie als Plattform für einen biologischen Test dienen. Der Redox-Mediator wird hierbei z.B. durch eine Enzymmarkierung positionsspezifisch generiert bzw. in seine elektrochemisch aktive Form umgewandelt.

Auch die direkte Umgebung der Elektroden / Messpositionen kann, wie ebenfalls bereits erwähnt, so ausgestaltet sein, dass sie in der Lage ist, Fängermoleküle zu binden. Hierfür eignen sich beispielsweise Materialien wie Oxide mit Hydroxid-Oberflächen oder modifizierte Silane/Siloxane oder anorganisch/organische Hybridmaterialien wie die siliciumhaltigen Ormocere®. Diese lassen sich auf ihrer Oberfläche leicht mit für die vorliegende Erfindung geeigneten Gruppen ausgestalten oder modifizieren, z.B. mit Aminen, Hydroxygruppen, Carboxylgruppen. In den vorgenannten Fällen wird man in der Regel von einer Anbindung von Fängermolekülen auf der/den Elektroden absehen, muss dies aber nicht.

Die direkte Umgebung der Elektroden / Messpositionen kann alternativ oder zusätzlich auch hydrophob ausgestaltet sein, um den Randwinkel von auf Elektrodenmaterial aufgebrachten Tröpfchen (insbesondere zur Anbindung von Fängermolekülen) möglichst groß zu halten. Stattdessen oder zusätzlich können die Elektroden / Messpositionen auch mit Ringen oder Stegen umgeben sein, die ebenfalls ein Verlaufen der Fängermaterial-Tröpfchen vermeiden helfen. Eine solche Ausgestaltung ist beispielsweise in DE 199 16 867 A1 beschrieben. Die Ringe oder Stege können bleibend oder intermediär vorgesehen sein. Dieselbe Druckschrift beschreibt auch die Verwendung von Stempeln für das Aufbringen von Fängermolekülen und den Aufbau von Mikrokapillarreaktoren; Ausgestaltungen, die sich gleichermaßen für die vorliegende Erfindung eignen.

Die Elektroden / Messpositionen können auf einem beliebigen Trägermaterial angeordnet sein, beispielsweise auf einem organischen Substrat wie Kunststoff, aber auch auf Leiterplatten oder dgl.. Gegebenenfalls kann selbstverständlich auch ein Siliziumwafer oder dgl. als Träger dienen, auch wenn dies in der Regel nicht notwendig sein wird. Günstig ist die Kombination von Goldelektroden und einem Kunststoffsubstrat. Die Elektroden können in einfacher Weise auf das Substrat aufgebracht sein, desgleichen die Leiterstrukturen. Die Leiterstrukturen können aus Gold, aber - z.B. um Kosten zu sparen - auch aus Kupfer oder Aluminium oder einem anderen gängigen Material bestehen.

Die Gestalt der Elektroden / Messpositionen ist unkritisch, weshalb sie eine beliebige Form aufweisen können. Sie können beispielsweise flächig sein und eine runde, ovale, rechteckige, länglich- gestreckte, quadratische oder sonstige mehreckige Form besitzen, je nach Anforderung an den Test und die sonstigen Bedingungen wie Anordnung eines oder mehrerer Fluidkanäle. Sie können durchgehend sein oder Aussparungen aufweisen, deren Oberfläche beispielsweise wie oben beschrieben zur Anbindung von Fängermaterialien geeignet ist. Sie können bei Bedarf in das umgebende Substrat eingebettet sein, beispielsweise zur Verbesserung der Strömungsverhältnisse im Fluidkanal, in dem sie sich befinden. Alternativ können sie auf das Substrat aufgebracht, beispielsweise aufgedampft, gedruckt, aufplattiert, aufgelötet oder in sonstiger Weise aufgebracht sein. Diese Varianten sind gegenüber der Einbettung deutlich kostengünstiger. Die Elektroden / Messpositionen können eine beliebige Größe besitzen. Um eine Vielzahl von Elektroden gleichzeitig ansteuern und vermessen zu können, beispielsweise auf einem Chip, ist es jedoch günstig, die Fläche der Elektroden relativ klein auszugestalten, z.B. mit einer Oberfläche von ca. 0.05 bis 0.5 mm². Die Elektroden können dabei ggf. als Mikroelektroden oder Ultramikroelektroden (mit Längen- und/oder Breitenabmessungen im µm- oder Sub-µm-Bereich) ausgestaltet sein. Schließlich ist anzumerken, dass die Elektroden / Messpositionen gegebenenfalls auch eine dreidimensionale Struktur aufweisen, z.B. als Tropfen, Lötbump, Draht oder Plättchen ausgestaltet sein können.

Das Verfahren benötigt typischerweise eine Referenzelektrode, die sich jedoch nicht auf demselben Substrat wie die Messelektrode(n) befinden muss. Es ist ausreichend, wenn sich die Referenzelektrode über das Detektionsfluid (z.B. Puffer mit oder ohne Redox-Species) in leitender Verbindung zu den Messelektroden befindet. Die Referenzelektrode wird nicht mit Strom beaufschlagt. Sie besteht aus einem für das jeweilige Verfahren geeigneten Material, beispielsweise Iridium/Iridiumoxid, Kalomel, Ag/AgCl. Für das Verfahren wird weiterhin eine Gegenelektrode benötigt, die zum Aufbringen des gewünschten Stroms auf die Messelektroden auf einen entsprechenden Gegenwert aufgeladen wird. Auch diese Elektrode muss sich nicht notwendigerweise auf demselben Substrat befinden wie die Messelektroden. Sie besteht typischerweise - aber nicht zwingend - aus Gold.

Der Vorteil des erfindungsgemäßen Verfahrens besteht darin, dass eine elektrische Auslesung auf in ihrer Form beliebig gestalteten Elektroden mittels Redox-Cycling erfolgen kann, deren Sensitivität der einer Auslesung auf interdigitalen Elektrodenstrukturen vergleichbar ist. Die Herstellung der Elektroden kann bei Vermeidung digitaler Strukturen deutlich einfacher und kostengünstiger erfolgen, da auf teure Halbleiter- und Beschichtungstechniken mit Masken, Polymerauftrag, Belichtungen und Auswaschungen für die benötigten Sub-µm-Elektrodenstrukturen für das herkömmliche Redox-Cycling verzichtet werden kann. Außerdem steigt die Produktions-Ausbeute dieser Elektroden, da kein Ausschuss durch elektrische Kurzschlüsse entstehen kann, die bei den herkömmlichen Sub-µm-Interdigitalstrukturen z.B. durch die versehentliche Anlagerung von Partikeln aus den Reinraumprozessen entstehen können.

Das erfindungsgemäße Verfahren wird vorzugsweise für die sogenannte Chip-Technologie eingesetzt. Hierbei befinden sich eine, vorzugsweise mehrere Messpositionen in Form von Elektroden auf einem Chip, der beispielsweise die Größe von etwa 50 bis 200 mm² aufweist. Jede Messposition ist über einen Fluidkanal mit einem Detektionsfluid oder einem Vergleichsfluid, z.B. reinem Puffer, beaufschlagbar. Die Referenzelektrode und die Gegenelektrode können, müssen aber nicht, ebenfalls auf dem Chip angeordnet sein. Alle Elektroden auf dem Chip sind mit elektrischen Leiterstrukturen für das Beaufschlagen mit den Potentialen und das Auslesen der Stromveränderungen verbunden.

Die elektrische Ansteuerung kann über einen separaten Potentiostaten erfolgen, sie kann aber auch als elektronisches Bauteil auf einem separaten Chip (2-Chip-Lösung) angeordnet sein. Dieser Chip wird mehrfach verwendet, während der Chip mit den Messpositionen in der Regel nur einmal verwendet und dann verworfen wird, insbesondere, wenn die Elektroden oder deren Umgebung mit Fängermaterial belegt wurden. Schließlich ist es auch möglich, die Elektronik direkt in dem Chip anzuordnen, der auch die Messpositionen trägt. All diese Varianten sind aus dem Stand der Technik bekannt, siehe beispielsweise E. Nebling et al., "Electrical Detection of Viral DNA Using Ultramicroelectrode Arrays", Anal. Chem. (2004), 76(3): 689-696, J. Albers et al., "Electrical biochip technology - a tool for microarrays and continuous monitoring", Anal Bioanal. Chem. (2003), 377(3): 521-7 oder R. Hintsche et al., "Fully electrical microarrays", Electrochemistry of Nucleic acids and Proteins, Toward Electrochemical Sensors for Genomics and Proteomics, 247-277, Herausgeber E. Palecek, F. Scheller und J. Wang, Elsevier, Amsterdam, 2005.

Die Erfindung soll nachstehend anhand von Ausführungsbeispielen näher erläutert werden.

### Beispiel 1

Auf einem Trägermaterial, beispielsweise passiviertem Silizium, sind mehrere Goldelektroden (Durchmesser 0,5 mm), eine Gegenelektrode und eine Iridium/Iridiumoxid-Referenzelektrode aufgedampft. Jede Goldelektrode entspricht hierbei einer Messposition. Auf die Elektroden 1 - 8 ist ein unspezifisches Protein (BSA) durch Adsorption gebunden. Auf den Elektroden 9 - 12 ist das Enzym β-Galaktosidase durch Adsorption gebunden. Die Goldelektroden können mittels einer mikrofluidischen Durchflusszelle, verbunden mit einer Mikropumpe, Schläuchen und einem Verteilerventil mit diversen flüssigen Medien überspült werden.

Die 12 Goldelektroden werden mit einem 16-Kanal Multipotentiostaten mit den benötigten Potentialen versorgt und die resultierenden Ströme im nano-Ampere-Bereich ausgelesen. Dieser Potentiostat besteht aus 2 8-fach Multiplexern, die die Elektroden nach erfolgter Aufgabe der jeweiligen Potentiale in kurzer Zeit sequenziell auslesen. An jeder Elektrode wird zwischen den Potentialen von +200 mV und -350 mV mit 1,0 Hertz kontinuierlich hin- und hergeschaltet. Als Bezug dient die Iridium/Iridiumoxid-Referenzelektrode. Die Gegenelektrode leitet Differenzströme ab. die Redoxpotentiale des Mediators p-Aminophenol liegen innerhalb des Bereiches von +200 mV und -350 mV.

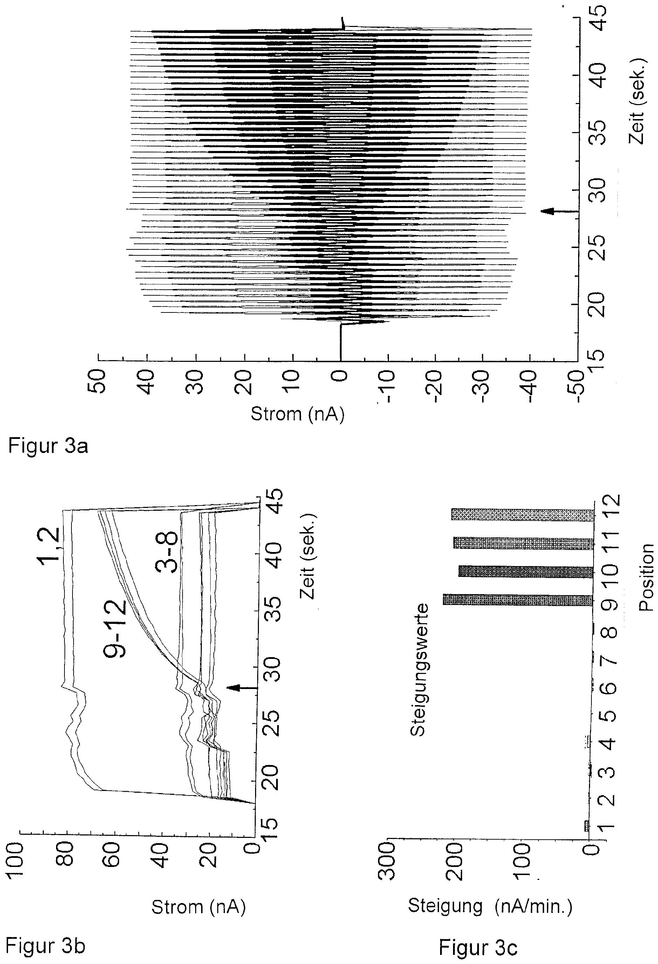

Nach dem Waschschritt mit Puffer (PBS pH: 7,0) wird ein Enzymsubstrat gelöst in Puffer (1,0 mg/ml in PBS pH: 7,0) über die Elektroden gegeben. Dieses Substrat ist p-Aminophenyl-β-D-Galactopyranosid (pAP-β-gal) und wird durch das Enzym β-Galaktosidase von einer elektrochemisch inaktiven Form in den elektrochemisch aktiven Redox-Mediator p-Aminophenol überführt. Während der elektrochemischen Messung wird der Flüssigkeitsstrom gestoppt. Als Resultat wird an jeder Goldelektrode unabhängig von den anderen je nach Anwesenheit oder Abwesenheit des Enzyms ein Redox-Strom generiert oder nicht. Dieser Strom steigt an den Enzym tragenden Elektroden über die Zeit kontinuierlich an, da das Enzym stetig das Substrat umsetzt und so den Redox-Mediator freisetzt. Man erwartet demnach an den Elektroden 1 - 8 keinen Anstieg des Stroms über die Zeit (Vergleichselektroden) und an den Elektroden 9 - 12 einen Strom-Anstieg über die Zeit (Messelektroden). Die hierbei verwendete neuartige elektrochemische Auslesung ist in **Figur 3** gezeigt.

**Figur 3a** zeigt die parallele Strom-Messung über die Zeit an jeder einzelnen von 12 Goldelektroden mit Redox-Cycling auf jeder Elektrode anhand der resultierenden, übereinander liegenden Messwerte der Stromsignale für alle Elektroden. Gemessen wird der Umsatz von p-Aminophenol individuell an jeder Elektrode (+200 mV, -350 mV mit 1,0 Hz). Nachdem das gelöste Substrat die Goldelektroden vollständig überspült hat, wird der Flüssigkeitsstrom gestoppt (Sekunde 28, siehe Pfeil), die Messung des Stroms läuft jedoch weiter. Je nach angelegtem Potential erhält man einen Oxidationsstrom-Peak (positiv) oder einen Reduktionsstrom-Peak (negativ). Die Hauptkomponente ist der kapazitive Umladestrom, der jedoch bei der Auswertung später herausgemittelt wird.

**Figur 3b** zeigt die Summe der Stromsignal-Beträge aus Oxidations- und Reduktionsstrom für jede einzelne Elektrode (Pfeil: Stoppen des Flüssigkeitsstroms). Die Positionen 1 - 8 zeigen einen weitestgehenden waagerechten Verlauf der Stromsignale. Die Positionen 9 - 12 zeigen hingegen einen Anstieg des Stroms über die Zeit nach Abschalten des Flüssigkeitsstroms (Pfeil). Da bei den Elektroden 1 - 8 das Enzym fehlt, wird hier kein aktiver Redox-Mediator gebildet und es resultieren somit keine Redox-Ströme. Auf den Elektroden 9 - 12 generiert das Enzym den aktiven Redox-Mediator, und dadurch steigt der Strom an diesen Positionen über die Zeit kontinuierlich an. Die Elektroden 1 - 8 zeigen einen unterschiedlich hohen, nahezu konstanten Strom. Dieses ist der kapazitive Umladestrom, der nach jeder Umladung zwar schnell im Millisekundenbereich abklingt, seine absolute Höhe pro Position bleibt aber bei jedem Messvorgang nahezu identisch. Die unterschiedliche Höhe dieses Umladestroms von Elektrode zu Elektrode resultiert daraus, dass die Potentiale an jeder Elektrode gleichzeitig umgeschaltet werden, die Auslesungen durch die Multiplexer aber leicht zeitversetzt sind. An den Elektroden, die kurz nach dem Umschalten gemessen werden (1, 2). ist dieser Umladestrom noch hoch, da seine Relaxation noch nicht abgeschlossen ist.

**Figur 3c** zeigt die Auftragung der Änderung der Stromwerte über die Zeit (StromSteigung) für jede Position individuell als Balkengrafik. Die waagerecht verlaufenden Ströme der "negativen" Elektroden 1 - 8 zeigen hier unabhängig von ihrer Höhe (Figur 3b) nahezu keine Veränderung. Der Strom an den "positiven" Positionen 9 - 12 hingegen steigt über die Zeit deutlich an. Dies zeigt eindeutig die Anwesenheit des Enzyms. Der Mittelwert der Stromsteigung der Positionen 9 - 12 beträgt etwa 209 nA/min.

Das Redox-Cycling auf Interdigitalelektroden ergibt bei vergleichbarer Versuchsanordnung eine Stromsteigung von etwa 300 nA/min. Misst man nur die Oxidation des Mediators bei +200 mV, ergibt sich eine Stromsteigung von etwa 20 nA/min.

Die oben beschriebene Erfindung ist somit zur sensitiven elektrischen Auslesung von Redox-Mediatoren auf nicht interdigitalen Elektroden geeignet und ist deutlich empfindlicher als nur die Oxidation auf solchen Elektroden, während die Sensitivität der Auslesung derjenigen auf interdigitalen Elektroden vergleichbar ist.

### Beispiel 2

Zur Durchführung eines biologischen Tests werden die Goldelektroden zuerst mit geeigneten Fängermolekülen adsorptiv belegt. Ein spezifisch an diesen Fängermolekülen gebundenes Zielmolekül wird durch ein Enzym markiert (z.B. β-Galaktosidase). Das zugeführte Substrat wird durch dieses Enzym in den aktiven Redox-Mediator umgesetzt. Dieser kann elektrodenspezifisch mit der oben beschriebenen Methode sensitiv gemessen werden.

## Patentansprüche

1. Verfahren zum Detektieren einer oder mehrerer chemischer oder biologischer Species, die entweder eine Redox-Reaktion eingehen können oder direkt oder indirekt ein Molekül generieren können, das eine Redox-Reaktion eingehen kann, wobei durch die genannte Redox-Reaktion generierter Strom an mindestens einer Elektrode detektiert wird,
umfassend die folgenden Schritte,
- Positionieren einer zeitlich variierenden Menge der Species oder des Moleküls auf, an oder in der Nähe der mindestens einen Elektrode innerhalb eines Zeitraums t₁-t₂,
- mehrfaches Hin- und Herschalten der mindestens einen Elektrode während des Zeitraums t₁-t₂ zwischen zwei unterschiedlichen Potentialen derart, dass sie gegenüber einer Referenzelektrode Potentiale einnimmt, die im Bereich des Oxidationspotentials der genannten Species oder des genannten Moleküls oder darüber bzw. im Bereich des Reduktionspotentials der genannten Species oder des genannten Moleküls oder darunter liegen, wodurch die genannte Species / das genannte Molekül abwechselnd reduziert und oxidiert wird,
- Erfassen des über den Zeitraum t₁-t₂ durch wiederholte Reduktion und Oxidation der Species / des Moleküls in der mindestens einen Elektrode gebildeten Stroms und Auswerten der zeitlichen Veränderung der Summe aus den Beträgen von Oxidations- und Reduktionsstrom.

2. Verfahren nach Anspruch 1, **dadurch gekennzeichnet, dass** man für eine quantitative Detektion der Species die Steigung der Stromwerte im Bereich t₁-t₂ oder das unter den Stromwerten liegende Integral misst und mit Referenzwerten vergleicht.

3. Verfahren nach einem der voranstehenden Ansprüche, **dadurch gekennzeichnet, dass** das mehrfache Hin- und Herschalten mit einer Frequenz im Bereich von 0,1 bis 10 Hz erfolgt und/oder dass die Anzahl der Schaltvorgänge im Zeitintervall t₁-t₂ zwischen 10 und 100 und vorzugsweise zwischen 15 und 50 liegt.

4. Verfahren nach einem der voranstehenden Ansprüche, wobei die eine oder mehreren Species eine Redox-Reaktion eingehen kann/können, **dadurch gekennzeichnet, dass** das Positionieren der Species das Überleiten eines die Species enthaltenden Fluids über die mindestens eine Elektrode umfasst.

5. Verfahren nach einem der Ansprüche 1 bis 3, wobei die eine oder mehreren Species eine Redox-Reaktion eingehen kann/können, **dadurch gekennzeichnet, dass** das Positionieren der Species die folgenden Schritte umfasst:
- Das Bereitstellen von Fängermolekülen auf der mindestens einen Elektrode oder in der unmittelbaren Umgebung der Elektrode,
- Das Überleiten eines die Species enthaltenden Fluids über die mindestens eine Elektrode oder deren unmittelbare Umgebung derart, dass die Fängermoleküle die Species durch Anbinden oder Komplexieren einfangen.

6. Verfahren nach einem der Ansprüche 1 bis 3, wobei die eine oder mehreren Species direkt oder indirekt ein Molekül generieren kann/können, das eine Redox-Reaktion eingehen kann, **dadurch gekennzeichnet, dass** das Positionieren des Moleküls die folgenden Schritte umfasst:
- Das Überleiten eines die Species enthaltenden Fluids über die mindestens eine Elektrode oder deren unmittelbare Umgebung derart, dass die Species an der mindestens einen Elektrode oder in unmittelbarer Umgebung der Elektrode gebunden wird,
- Das Generieren des Moleküls, das eine Redox-Reaktion eingehen kann, durch die Species,
wobei auf der mindestens einen Elektrode und/oder deren unmittelbarer Umgebung Fängermoleküle angebracht sind, die in der Lage sind, die Species durch Anbinden oder Komplexieren einzufangen.

7. Verfahren nach Anspruch 6, **dadurch gekennzeichnet, dass** zum Generieren des Moleküls, das eine Redox-Reaktion eingehen kann, ein Fluid über die mindestens eine Elektrode und ggf. ihre unmittelbare Umgebung geleitet wird, das ein Vorläufer-Molekül enthält, aus dem die Species das genannte Molekül generieren kann, das eine Redox-Reaktion eingehen kann.

8. Verfahren nach einem der voranstehenden Ansprüche, **dadurch gekennzeichnet, dass** mindestens zwei Elektroden vorhanden sind, die mehrfach während des Zeitraums t₁-t₂ zwischen den genannten unterschiedlichen Potentialen derart gleichsinnig hin- und hergeschaltet werden, dass sie gegenüber einer Referenzelektrode Potentiale einnehmen, die im Bereich bzw. unter/über dem Redoxpotential der genannte Species oder des genannten Moleküls liegen, wodurch die genannte Species / das genannte Molekül gleichzeitig an jeder der mindestens zwei Elektroden abwechselnd reduziert und oxidiert wird.

9. Verfahren nach Anspruch 8, **dadurch gekennzeichnet, dass** die beiden oder zwei der Elektroden mit unterschiedlichen Fängermolekülen belegt sind, die unterschiedliche Species binden oder detektieren können.

10. Vorrichtung zum Durchführen eines Verfahrens nach einem der voranstehenden Ansprüche, umfassend ein Elektrodenarray mit mindestens einer Messposition und mindestens einer Messelektrode pro Messposition und einer Referenzelektrode, das derart ausgebildet ist, dass alle Messelektroden gegenüber der Referenzelektrode gleichsinnig abwechselnd als Kathode und als Anode geschaltet werden können, eine Gegenelektrode, eine Referenzelektrode sowie eine Schaltungsanordnung, die die Messelektrode(n) gegenüber der Referenzelektrode gleichsinnig abwechselnd als Kathode und als Anode schalten kann, und
Mittel zum Erfassen des durch wiederholte Reduktion und Oxidation an den Messelektroden gebildeten Stroms und zum Auswerten der zeitlich Veränderung der Summe aus den Beträgen von Oxidations- und Reduktionsstrom.

11. Vorrichtung nach Anspruch 10, umfassend ein Substrat in Chip-Format und eine oder mehrere Messelektroden, die aus Gold oder Platin bestehen oder mit Gold oder Platin beschichtet sind.

12. Vorrichtung nach Anspruch 10 oder 11, worin die Messelektroden des Elektrodenarrays Abmessungen im µm-Bereich oder unterhalb des µm-Bereichs aufweisen.

13. Vorrichtung nach Anspruch 12, **dadurch gekennzeichnet, dass** die Schaltungsanordnung ein Potentiostat ist.

14. Vorrichtung nach Anspruch 10, **dadurch gekennzeichnet, dass** die Schaltungsanordnung eine elektronische Schaltungsanordnung ist, die sich entweder auf oder in einem Substrat befindet, das nicht das Substrat für die Messelektroden ist, oder die sich auf oder in demselben Substrat befindet, das die Messelektroden trägt.

## Claims

1. A process for detecting one or more chemical or biological species, which either can react in a redox reaction or can directly or indirectly generate a molecule capable of reacting in a redox reaction, wherein current generated by said redox reaction is detected at at least one electrode,
comprising the following steps:
- positioning a quantity of the species or the molecule, which quantity varies over time, at or in the proximity of the at least one electrode within a period t₁-t₂,
- multiple switching back and forth of the at least one electrode during the period t₁-t₂ between two different potentials such that the electrode, relative to a reference electrode, adopts potentials that are within or above the range of the oxidation potential of said species or of said molecule, or within or below the range of the reduction potential of said species or of said molecule, whereby said species/said molecule is alternatingly reduced and oxidized, and
- detecting the current generated in the at least one electrode by the repeated reduction and oxidation of the species/molecule over the period t₁-t₂ and evaluating the change over time of the sum of the values of the oxidation current and the reduction current.

2. A process according to claim 1, **characterized in that**, for a quantitative detection of the species, the slope/increase of the current values in the range of t₁-t₂ or the integral below the current values is measured and is compared with reference values.

3. A process according to one of the preceding claims, **characterized in that** the multiple switching back and forth is carried out with a frequency of 0.1 to 10 Hz and/or the number of switching operations in the time interval t₁-t₂ is between 10 and 100, preferably between 15 and 50.

4. A process according to one of the preceding claims, wherein the one or more species can react in a redox reaction, **characterized in that** the positioning of the species comprises passing a fluid containing the species over the at least one electrode.

5. A process according to one of claims 1 to 3, wherein the one or
more species can react in a redox reaction, **characterized in that** the positioning of the species comprises the following steps:
- providing scavenger molecules on the at least one electrode or in the immediate vicinity of the electrode, and
- passing a fluid containing the species over the at least one electrode or its immediate vicinity, such that the scavenger molecules capture the species by bonding or complexing.

6. A process according to one of claims 1 to 3, wherein the one or
more species can directly or indirectly generate a molecule capable of reacting in a redox reaction, **characterized in that** the positioning of the species comprises the following steps:
- passing a fluid containing the species over the at least one electrode or its immediate environment, such that the species is bound at the at least one electrode or in the immediate vicinity of the electrode,
- generating the molecule capable of reacting in a redox reaction, by the species,
wherein on the at least one electrode and/or its immediate vicinity, scavenger molecules are arranged which are capable of capturing the species by bonding or complexing.

7. A process according to claim 6, **characterized in that** to generate the molecule capable of reacting in a redox reaction, a fluid containing a precursor molecule is passed over the at least one electrode and optionally the immediate vicinity thereof, from which the species can generate the molecule capable of reacting in a redox reaction.

8. A process according to one of the preceding claims, **characterized in that** at least two electrodes are present which are repeatedly switched back and forth in the same direction between said different potentials within the period t₁-t₂ in such a manner that they adopt potentials with respect a reference electrode that are in the range of or above/below the redox potential of said species or of said molecule, whereby said species/said molecule is simultaneously reduced and oxidized at each of the at least two electrodes.

9. A process according to claim 8, **characterized in that** the two electrodes or two of the electrodes are coated with different scavenger molecules which can bind or detect different species.

10. A device for carrying out the process according to one of the preceding claims, comprising an electrode array having at least one measuring position and at least one measuring electrode per measuring position and a reference electrode, which (array) is designed such that all measuring electrodes can be switched alternatingly in the same direction as a cathode and as an anode relative to the reference electrode, a counter electrode, a reference electrode, and a switching device which can switch the measuring electrode(s) alternatingly as a cathode and as an anode in the same direction relative to the reference electrode, and means for detecting the current generated at the measuring electrodes by the repeated reduction and oxidation and for evaluating the change over time of the sum of the absolute values of the oxidation current and the reduction current.

11. A device according to claim 10, comprising a substrate in chip format and one or more measuring electrodes which consist of gold or platinum or are coated with gold or platinum.

12. A device according to claim 10 or 11, wherein the measuring electrodes of the electrode array have dimensions in the µm range or below the µm range.

13. A device according to claim 12, **characterized in that** the switching device is a potentiostat.

14. A device according to claim 10, **characterized in that** the switching device is an electronic switching device which is located on or in a substrate which is not the substrate for the measuring electrodes, or which is located on or in the same substrate which carries the measuring electrodes.

## Revendications

1. Procédé de détection d'une ou de plusieurs espèce(s) chimique(s) ou biologique(s), apte(s) à subir une réaction redox ou pouvant générer directement ou indirectement une molécule apte à subir une réaction redox, dans lequel on détecte par au moins une électrode le courant généré par ladite réaction redox, comprenant les étapes suivantes:
- positionnement d'une quantité, variant dans le temps, de l'espèce ou de la molécule, contre ou au voisinage de ladite au moins une électrode à l'intérieur d'un intervalle de temps t₁-t₂,
- commutation répétée de ladite au moins une électrode pendant l'intervalle de temps t₁-t₂ entre deux potentiels différents, de telle manière que l'électrode prenne par rapport à une électrode de référence des potentiels, qui se trouvent dans la zone du potentiel d'oxydation des espèces mentionnées ou de la molécule mentionnée ou au-delà, ou dans la zone du potentiel de réduction des espèces mentionnées ou de la molécule mentionnée ou en-deçà, ce qui va provoquer en alternance une réduction et une oxydation des espèces mentionnées/de la molécule mentionnée,
- détection du courant formé dans ladite au moins une électrode par la réduction et l'oxydation répétées des espèces/de la molécule pendant l'intervalle de temps t₁-t₂, et évaluation de la variation temporelle de la somme des valeurs des courants d'oxydation et de réduction.

2. Procédé selon la revendication 1, **caractérisé en ce que**, pour une détection quantitative des espèces, on mesure la pente des valeurs de courant dans la zone t₁-t₂ ou l'intégrale située sous les valeurs de courant et on la compare avec des valeurs de référence.

3. Procédé selon l'une quelconque des revendications précédentes, **caractérisé en ce que** l'on effectue la commutation répétée avec une fréquence située dans la plage de 0,1 à 10 Hz et/ou **en ce que** le nombre des opérations de commutation dans l'intervalle de temps t₁-t₂ vaut entre 10 et 100 et de préférence entre 15 et 50.

4. Procédé selon l'une quelconque des revendications précédentes, dans lequel ladite une ou lesdites plusieurs espèce (s) est/sont apte (s) à subir une réaction redox, **caractérisé en ce que** le positionnement de l'espèce comprend le passage d'un fluide contenant l'espèce sur ladite au moins une électrode.

5. Procédé selon l'une quelconque des revendications 1 à 3, dans lequel ladite une ou lesdites plusieurs espèce(s) est/sont apte (s) à subir une réaction redox, **caractérisé en ce que** le positionnement de l'espèce comprend les étapes suivantes:
- préparation de molécules de capture sur ladite au moins une électrode ou au voisinage immédiat de l'électrode,
- passage d'un fluide contenant l'espèce sur ladite au moins une électrode ou sur son voisinage immédiat, de telle manière que les molécules de capture capturent l'espèce par liaison ou complexation.

6. Procédé selon l'une quelconque des revendications 1 à 3, dans lequel ladite une ou lesdites plusieurs espèce(s) est/sont apte(s) à générer directement ou indirectement une molécule, qui est apte à subir une réaction redox, **caractérisé en ce que** le positionnement de la molécule comprend les étapes suivantes:
- passage d'un fluide contenant l'espèce sur ladite au moins une électrode ou sur son voisinage immédiat, de telle manière que l'espèce soit liée à ladite au moins une électrode ou dans le voisinage immédiat de l'électrode,
- génération de la molécule, qui est apte à subir une réaction redox, par l'espèce,
dans lequel des molécules de capture, qui sont capables de capturer l'espèce par liaison ou complexation, sont apportées sur ladite au moins une électrode et/ou dans son voisinage immédiat.

7. Procédé selon la revendication 6, **caractérisé en ce que**, pour générer la molécule qui est apte à subir une réaction redox, on fait passer sur ladite au moins une électrode et éventuellement dans son voisinage immédiat, un fluide qui contient une molécule de précurseur, à partir de laquelle l'espèce peut générer ladite molécule, qui est apte à subir une réaction redox.

8. Procédé selon l'une quelconque des revendications précédentes, **caractérisé en ce qu'**il se trouve au moins deux électrodes, qui sont commutées de façon répétée dans le même sens pendant l'intervalle de temps t₁-t₂ entre les potentiels différents précités, de telle manière qu'elles prennent par rapport à une électrode de référence des potentiels qui se trouvent dans la zone ou en dessous/au-dessus du potentiel redox de l'espèce mentionnée ou de la molécule mentionnée, ce qui va provoquer en alternance, simultanément sur chacune desdites au moins deux électrodes, une réduction et une oxydation de l'espèce mentionnée/de la molécule mentionnée.

9. Procédé selon la revendication 8, **caractérisé en ce que** les deux ou deux des électrodes sont garnies de molécules de capture différentes, qui sont aptes à lier ou à détecter des espèces différentes.

10. Dispositif permettant la mise en oeuvre d'un procédé selon l'une quelconque des revendications précédentes, comprenant un réseau d'électrodes avec au moins une position de mesure et au moins une électrode de mesure par position de mesure et une électrode de référence, qui est configuré de telle manière que toutes les électrodes de mesure puissent être, par rapport à l'électrode de référence, montées en alternance dans le même sens en tant que cathode et en tant qu'anode, ainsi qu'un agencement de circuit, qui peut commuter la/les électrode(s) de mesure par rapport à l'électrode de référence en alternance dans le même sens en tant que cathode et en tant qu'anode, et des moyens pour détecter le courant généré aux électrodes de mesure par la réduction et l'oxydation répétées et pour évaluer la variation temporelle de la somme des valeurs des courants d'oxydation et de réduction.

11. Dispositif selon la revendication 10, comprenant un substrat au format de puce et une ou plusieurs électrodes de mesure, qui sont constituées d'or ou de platine ou qui sont revêtues d'or ou de platine.

12. Dispositif selon la revendication 10 ou 11, dans lequel les électrodes de mesure du réseau d'électrodes présentent des dimensions dans le domaine du micromètre ou inférieures au domaine du micromètre.

13. Dispositif selon la revendication 12, **caractérisé en ce que** l'agencement de circuit est un potentiostat.

14. Dispositif selon la revendication 10, **caractérisé en ce que** l'agencement de circuit est un agencement de circuit électronique, qui se trouve sur ou dans un substrat, qui n'est pas le substrat destiné aux électrodes de mesure, ou qui se trouve sur ou dans le même substrat qui porte aussi les électrodes de mesure.
